# EUROPEAN PATENT SPECIFICATION

(11) **EP 1 216 063 B1**
(45) Date of publication and mention of the grant of the patent: **28.05.2003**
(21) Application number: 00964783.5
(22) Date of filing: 15.09.2000
(51) Int. Cl.: A61L 9/03

(54) **AIR FRESHENING APPARATUS**
VORRICHTUNG ZUR LUFTVERBESSERUNG
DISPOSITIF DESODORISANT

(30) Priority: 16.09.1999 EP 99203023
(43) Date of publication of application: 26.06.2002
(73) Proprietor: Sara Lee/DE N.V., 3532 AA Utrecht (NL)
(72) Inventor: KUHN, Petrus, Henricus, Aloysius, Nicolaas, NL-2565 HG The Hague (NL)
(74) Representative: Prins, Adrianus Willem
(86) International application number: NL0000654
(87) International publication number: WO01019418

(56) References cited:
- WO-A-99/22776
- DE-C- 943 680
- FR-A- 1 040 409
- FR-A- 1 139 960
- FR-A- 2 510 410
- US-A- 5 840 257
- US-A- 5 891 400

## Description

The present invention relates to an apparatus, comprising a reservoir with a wick, which reservoir can be filled with a fuel, particularly with lamp oil and comprising a separate holder for an active component.

Such an apparatus is known FR-A-1 139 960 which discloses an oil lamp having an reservoir for fuel in which a wick is provided. Above the wick a holder for a fumigation substance, e.g. to fumigate insects, is provided. The bottom of the holder is directly heated by the hot burning gases which rise from the flame.

FR-A-2 510 410 discloses a electric lamp bulp with a holder for perfume. Air, which is heated by the electric lamp bulp is led to a wick which is placed in the perfume which is in the holder. Consequently, the perfume is evaporated through the wick more efficiently by the heated air which is flowing along the wick.

US-A-5 891 400 shows a candle container with a surrounding open top container for receiving and holding a gel containing a vaporizable or diffusable substance that passes from the gel into the surrounding atmosphere after exposure to the thermal energy from the lighted candle.

Dutch patent application NL-A-10.06055 discloses an apparatus in which the reservoir is filled with a mixture of a lamp fuel and an active component, such as a fragrance, which mixture is absorbed by a wick. The upper part of the wick is surrounded by a heat conducting bushlike element with openings via which the fragrance evaporates during burning. A shield is present to prevent the evaporated fragrance entering into the flame and getting burned.

A disadvantage of a mixture of lamp fuel and fragrance is that the composition of the mixture will not be constant clue to a different evaporating rate and to a certain evaporating of the fragrance during the period that the apparatus is not in use, i.e. is not burning.

The purpose of the invention is to improve the known apparatus, particularly with respect to the above disadvantage. Therefore, according to the invention, the air freshening apparatus as described in the preamble is characterized in that the apparatus is an air freshening apparatus further comprising heat conducting means for heat transport from the wick, when burning, to the separate holder for the active component, such as a fragrance, the heat conducting means comprising a metal element which is mounted in the vicinity of the wick at such a distance that, when the wick is burning, the flame heats the metal element.

In a first preferred embodiment the reservoir is provided with a lid holding the wick and with a lamp glass thereabove while in the lamp glass there is mounted a metal element, which forms part of the heat conducting means. In this embodiment the metal element can be mounted in the lamp glass in the vicinity of, particularly above the location of the wick, when burning.

From a constructional point of view the holder can form an integral part of the apparatus. Also the holder can form an integral part of the lamp glass. Further at least part of the holder can form part of the heat conducting means.

In a further embodiment the holder is replaceable, while the apparatus is provided with connecting means to connect the holder to the apparatus. In that embodiment at least part of the connecting means or at least part of a contact surface between the holder and the apparatus can form part of the heat conducting means.

The holder can be provided with a porous wick to transport the active component out of the holder. Also the holder can be provided with one or more openings in the upper part thereof via which openings the active component can evaporate. In stead of a porous material, the wick, particularly the wick in the holder, can be formed by a capillary element. Further, the holder can also be provided with a liquid permeable closing element, via which the active component is received by a carrier element, which carrier element can form part of the heat conducting means and serves as an evaporator.

The invention not only relates to an air freshening apparatus, but also to a combination of the above described air freshening apparatus and a holder for an active component, such as a fragrance.

The invention also relates to a holder for an active component, such as a fragrance, for use in the above described air freshening apparatus.

The invention will now be described with reference to the enclosed drawing, in which:
Fig. 1 shows a first embodiment of the air freshening apparatus according to the invention; and
Fig. 2 shows a second embodiment of such an air freshening apparatus.

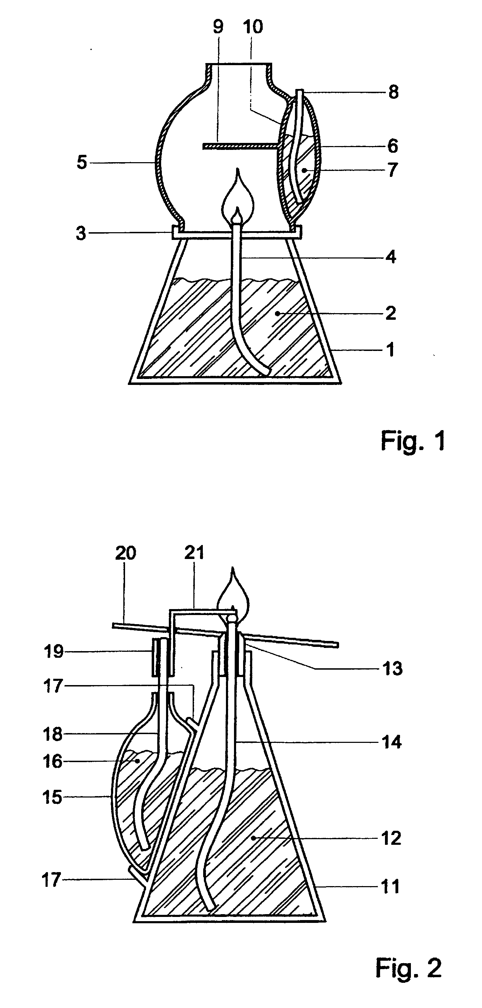

The first embodiment of the apparatus according to the invention as shown in fig. 1 comprises a reservoir 1, which is filled with lamp fuel 2 and is closed by a lid 3. Through an opening in the lid 3 a wick 4 is inserted into the lamp fuel in the reservoir 1. A lamp glass 5 is placed on the top of the lid 3. A separate holder 6, filled with an active component 7, such as a fragrance, is provided. In the embodiment shown, the holder 6 forms an integral part of the lamp glass 5. Through an opening in the upper part of the holder 6 a wick 8 is inserted into the active component 7 in the holder 6. In the lamp glass 5 a metal element 9 is mounted in the vicinity of the wick 4, particularly above the wick 4 at such a distance that, when the wick is burning, the flame heats the metal element 9. Heat conducting means are formed by the metal part 5 and at least part of the contact surface 10 between the holder 6 and the lamp glass 5. Particularly, part of the contact surface 10 can be a metal part connected to the metal element 9. When the wick 4 is burning, heat is conducted from the metal element 9 and at least part of the contact surface 10 to the active component 7 in the holder 6, with the consequence that the active component is evaporated via the wick 8.

Fig. 2 shows an embodiment of the apparatus according to the invention, in which a reservoir 11, filled with a lamp fuel 12 is closed by a heat conducting plug 13. Through an opening in the plug 13 a wick 14 is inserted. A separate holder 15, filled with an active component 16, such as a fragrance, is replaceable mounted on a side surface of the reservoir 11, e.g. by clamping means 17. Through the open end of the holder 15 a wick 18 is inserted. The wick 18 reaches out of a bushlike element 19, which is mounted above the opening of the holder 15. The bushlike element 19 is connected is provided with a projection 21, which reaches to above the plug 13 and forms with the plug 13 heat conducting means. When the wick 14 is burning, heat is conducted through the plug 13 and the bushlike element 19 to the active component 16 in the holder 15, with the consequence that the active component is evaporated via the wick 18. A shield 20 is provided to prevent the evaporated active component entering into the flame and getting burned.

It will be clear that the invention is not restricted to the preferred embodiments shown in the figures. Particularly, it will be clear to the skilled man that many alternatives exist for performing the function of the apparatus discussed. So, the form and size of the reservoir, the holder and the lamp can arbritray chosen. Taking into the heat generated by burning of lamp fuel several, preferably transparent materials can be used; however, the heat conducting means will be of metal.

## Claims

1. Air freshening apparatus comprising a reservoir with a wick, which reservoir can be filled with a fuel, particularly with lamp oil and comprising a separate holder for an active component, **characterized in that** the apparatus is an air freshening apparatus further comprising heat conducting means for heat transport from the wick, when burning, to the separate holder for the active component, such as a fragrance, the heat conducting means comprising a metal element which is mounted in the vicinity of the wick at such a distance that, when the wick is burning, the flame heats the metal element.

2. Air freshening apparatus according to claim 1, **characterized in that** the reservoir is provided with a lid holding the wick and with a lamp glass thereabove while in the lamp glass there is mounted a metal element, which forms part of the heat conducting means.

3. Air freshening apparatus according to claim 1 or 2, **characterized in that** the metal element is mounted in the lamp glass in the vicinity of, particularly above the location of the wick, when burning.

4. Air freshening apparatus according to any one of the preceding claims, **characterized in that** the holder forms an integral part of the apparatus.

5. Air freshening apparatus according to claim 2 and 4, **characterized in that** the holder forms an integral part of the lamp glass.

6. Air freshening apparatus according to any one of the preceding claims, **characterized in that** at least part of the holder forms part of the heat conducting means.

7. Air freshening apparatus according to anyone of the claims 1-3, **characterized in that** the holder is replaceable and that the apparatus is provided with connecting means to connect the holder to the apparatus.

8. Air freshening apparatus according to claim 7, **characterized in that** at least part of the connecting means or at least part of a contact surface between the holder and the apparatus forms part of the heat conducting means.

9. Air freshening apparatus according to any one of the preceding claims, **characterized in that** the holder is provided with a porous wick to transport the active component out of the holder.

10. Air freshening apparatus according to any one of the preceding claims 1-8, **characterized in that** the holder is provided with one or more openings in the upper part thereof via which openings the active component can evaporate.

11. Combination of an air freshening apparatus according to claim 7 or 8 and a holder for an active component, such as a fragrance.

12. Holder for an active component, such as a fragrance, for use in an air freshening apparatus according to claim 7 or 8.

## Patentansprüche

1. Eine Vorrichtung, die einen Behälter mit einem Docht beinhaltet, bei welcher der Behälter mit einem flüssigen Brennstoff gefüllt werden kann, vor allem mit Lampenöl, und welche ein separates Behältnis für einen Wirkstoff beinhaltet, ist dadurch charakterisiert, dass die Vorrichtung eine Vorrichtung zur Luftverbesserung ist, welche des weiteren über Mittel für die Wärmeleitung verfügt, die dazu dienen, Wärme vom Docht, wenn er brennt, zu dem separaten Behältnis für den Wirkstoff, wie zum Beispiel einem Duftstoff, zu leiten, wobei die Mittel für die Wärmeleitung ein Metallelement beinhalten, welches in der Nähe des Dochtes, in einem solchen Abstand angebracht ist, dass die Flamme das Metall erhitzt, wenn der Docht brennt.

2. Eine Vorrichtung zur Luftverbesserung gemäß Anspruch 1, welche dadurch charakterisiert ist, dass der Behälter mit einem Deckel, der den Docht hält, und einem, sich darüber befindlichen, Lampenglaskolben versehen ist, wobei in dem Lampenglaskolben ein Metallelement angebracht ist, welches einen Teil der Mittel für die Wärmeleitung bildet.

3. Eine Vorrichtung zur Luftverbesserung gemäß Anspruch 1 oder 2, welche dadurch charakterisiert ist, dass das Metallelement im Lampenglaskolben in der Nähe des Dochtes, genauer gesagt oberhalb von ihm, angebracht ist, wenn er brennt.

4. Eine Vorrichtung zur Luftverbesserung gemäß irgendeinem der vorausgegangenen Ansprüche, welche dadurch charakterisiert ist, dass das Behältnis ein integraler Bestandteil der Vorrichtung ist.

5. Eine Vorrichtung zur Luftverbesserung gemäß der Ansprüche 2 und 4, welche dadurch charakterisiert ist, dass das Behältnis ein integraler Bestandteil des Lampenglaskolbens ist.

6. Eine Vorrichtung zur Luftverbesserung gemäß irgendeinem der vorausgegangenen Ansprüche, welche dadurch charakterisiert ist, dass wenigstens ein Teil des Behältnisses einen Bestandteil der Mitteln für die Wärmeleitung bildet.

7. Eine Vorrichtung zur Luftverbesserung gemäß irgendeinem der Ansprüche 1 - 3, welche dadurch charakterisiert ist, dass das Behältnis auswechselbar ist, und dass die Vorrichtung mit Verbindungsstücken, die dazu dienen, das Behältnis mit der Vorrichtung zu verbinden, versehen ist.

8. Eine Vorrichtung zur Luftverbesserung gemäß Anspruch 7, welche dadurch charakterisiert ist, dass wenigstens ein Teil der Verbindungsstücke oder wenigstens ein Teil der Oberfläche, wo das Behältnis und die Vorrichtung Kontakt zueinander haben, einen Bestandteil der Mitteln für die Wärmeleitung bilden.

9. Eine Vorrichtung zur Luftverbesserung gemäß irgendeinem der vorausgegangenen Ansprüche, welche dadurch charakterisiert ist, dass das Behältnis über einen porösen Docht verfügt, um den Wirkstoff aus dem Behältnis zu leiten.

10. Eine Vorrichtung zur Luftverbesserung gemäß irgendeinem der vorausgegangenen Ansprüche 1 - 8, welche dadurch charakterisiert ist, dass das Behältnis in seinem oberen Bereich über eine oder mehrere Öffnungen verfügt, damit der Wirkstoff über diese Öffnungen verdampfen kann.

11. Eine Kombination einer Vorrichtung zur Luftverbesserung gemäß der Ansprüche 7 oder 8 und eines Behältnisses für einen Wirkstoff, wie etwa einem Duftstoff.

12. Ein Behältnis für einen Wirkstoff, wie etwa einem Duftstoff, um in einer Vorrichtung zur Luftverbesserung gemäß den Ansprüchen 7 oder 8 verwendet werden zu können.

## Revendications

1. Appareil comprenant un réservoir à mèche, le réservoir pouvant être rempli de combustible, en particulier d'huile pour lampe et comprenant un récipient séparé pour un composant actif, **caractérisé par le fait que** l'appareil est un appareil désodorisant comprenant en outre des moyens caloporteurs pour le transport de chaleur depuis la mèche, lorsqu'elle brûle, jusqu'au récipient séparé pour le composant actif, tel qu'un parfum, les moyens caloporteurs comprenant un élément métallique monté à proximité de la mèche à une distance telle que, lorsque la mèche brûle, la flamme chauffe l'élément métallique.

2. Appareil désodorisant selon la revendication 1, **caractérisé par le fait que** le réservoir comporte un couvercle tenant la mèche et un verre de lampe au-dessus et où dans le verre de lampe est monté un élément métallique faisant partie des moyens caloporteurs.

3. Appareil désodorisant selon l'une des revendications 1 et 2, **caractérisé par le fait que** l'élément métallique est monté dans le verre de lampe à proximité de, et en particulier au-dessus de, la position de la mèche, lorsqu'elle brûle.

4. Appareil désodorisant selon l'une des revendications précédentes, **caractérisé par le fait que** le récipient constitue une partie intégrante de l'appareil.

5. Appareil désodorisant selon les revendications 2 et 4, **caractérisé par le fait que** le récipient constitue une partie intégrante du verre de lampe.

6. Appareil désodorisant selon l'une quelconque des revendications précédentes, **caractérisé par le fait qu'**au moins une partie du récipient constitue une partie des moyens caloporteurs.

7. Appareil désodorisant selon l'une quelconque des revendications 1 à 3, **caractérisé par le fait que** le récipient est amovible et que l'appareil comporte des moyens de connexion pour connecter le récipient à l'appareil.

8. Appareil désodorisant selon la revendication 7, **caractérisé par le fait qu'**au moins une partie des moyens de connexion ou au moins une partie d'une surface de contact entre le récipient et l'appareil constitue une partie des moyens caloporteurs.

9. Appareil désodorisant selon l'une quelconque des revendications précédentes, **caractérisé par le fait que** le récipient comporte une mèche poreuse pour transporter le composant actif en dehors du récipient.

10. Appareil désodorisant selon l'une quelconque des revendications 1 à 8, **caractérisé par le fait que** le récipient comporte une ou plusieurs ouvertures dans sa partie supérieure, le composant actif pouvant s'évaporer à travers les dites ouvertures.

11. Combinaison d'un appareil désodorisant selon l'une des revendications 7 et 8 et d'un récipient pour un composant actif, tel qu'un parfum.

12. Récipient pour un composant actif, tel qu'un parfum, utilisable dans un appareil désodorisant selon l'une des revendications 7 et 8.
